(19) Europäisches Patentamt
European Patent Office
Office européen des brevets

(11) EP 4 230 188 A1

(12) # EUROPÄISCHE PATENTANMELDUNG

(43) Veröffentlichungstag:
**23.08.2023 Patentblatt 2023/34**

(21) Anmeldenummer: **23151937.2**

(22) Anmeldetag: **17.01.2023**

(51) Internationale Patentklassifikation (IPC):
***A61K 8/02*** (2006.01) ***A61K 8/19*** (2006.01)
***A61K 8/44*** (2006.01) ***A61K 8/46*** (2006.01)
***A61K 8/60*** (2006.01) ***A61K 8/73*** (2006.01)
***A61Q 5/02*** (2006.01) ***A61Q 5/12*** (2006.01)

(52) Gemeinsame Patentklassifikation (CPC):
**A61Q 5/12; A61K 8/0241; A61K 8/19; A61K 8/442; A61K 8/463; A61K 8/604; A61K 8/737; A61Q 5/02;**
A61K 2800/30; A61K 2800/34; A61K 2800/412; A61K 2800/596

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(30) Priorität: **14.02.2022 DE 102022201525**

(71) Anmelder: **Beiersdorf AG**
**20253 Hamburg (DE)**

(72) Erfinder:
- **Nemnich, Julia**
  **22589 Hamburg (DE)**
- **Reiter, Katharina**
  **21357 Barum (DE)**

(74) Vertreter: **Beiersdorf AG**
**Patentabteilung**
**Unnastraße 48**
**20245 Hamburg (DE)**

(54) **SHAMPOOZUBEREITUNG MIT DIAMANTPULVER**

(57) Die vorliegende Anmeldung betrifft ein kosmetisches Shampoo.



Processed by Luminess, 75001 PARIS (FR)

## Beschreibung

Kosmetische Produkte dienen im Allgemeinen nicht nur dazu, schön und attraktiv auszusehen, sondern sie tragen mit ihrer Wirkung entscheidend zu einem gesteigerten Selbstwertgefühl und zum Wohlbefinden der Menschen bei. Dementsprechend werden die verschiedensten kosmetischen Produkte zur täglichen Reinigung und Pflege der menschlichen Haut und Haar eingesetzt.

Bei der Körperpflege des Menschen spielt die Haarwäsche eine zentrale Rolle. Die Reinigung der Haare und der Kopfhaut von körpereigenem Fett, Hautabschilferungen, Schmutz und Gerüchen entspricht einem Grundbedürfnis des Menschen.

Zur Befriedigung dieses Bedürfnisses stand dem Menschen bis ins 20.Jahrhundert hinein allein Seife zur Verfügung, die aufgrund ihres alkalischen pH-Wertes für die Kopfhaut und die Augenschleimhäute wenig verträglich war und häufig Ablagerungen von Kalkseifen im Haar zurückließ. In den dreißiger Jahren des 20.Jahrhunderts erblickte das erste alkylsulfathaltige Shampoo das Licht der Welt. Seit Mitte der sechziger Jahre erobern Alkylethersulfate und andere Tenside den Shampoomarkt. Mit ihnen können die Nachteile von seifenhaltigen Zubereitungen vermieden werden. Heutzutage müssen moderne Shampoos das Haar nicht nur reinigen und gut verträglich sein. Vielmehr sollen sie das Haar auch pflegen und seine Frisierbarkeit und optische Attraktivität erhöhen.

Zur Befriedigung dieses Bedürfnisses stand dem Menschen bis ins 20.Jahrhundert hinein allein Seife zur Verfügung, die aufgrund ihres alkalischen pH-Wertes für die Kopfhaut und die Augenschleimhäute wenig verträglich war und häufig Ablagerungen von Kalkseifen im Haar zurückließ. In den dreißiger Jahren des 20.Jahrhunderts erblickte das erste alkylsulfathaltige Shampoo das Licht der Welt. Seit Mitte der sechziger Jahre erobern Alkylethersulfate und andere Tenside den Shampoomarkt. Mit ihnen können die Nachteile von seifenhaltigen Zubereitungen vermieden werden. Heutzutage müssen moderne Shampoos das Haar nicht nur reinigen und gut verträglich sein. Vielmehr sollen sie das Haar auch pflegen und seine Frisierbarkeit und optische Attraktivität erhöhen.

Haarshampoos enthalten eine Vielzahl unterschiedlicher Komponenten, um den einzelnen Anforderungen an das Produkt gerecht zu werden:
Die Reinigungskraft der Shampoos wird bewirkt durch die Anwesenheit von anionischen, amphoteren und nichtionischen Tensiden als oberflächenaktive Verbindungen in den Zubereitungen. Tenside sorgen darüber hinaus für das Schaumvermögen der Haarreinigungsmittel. Wichtig bei der Auswahl der Tenside ist darüber hinaus ihre Unempfindlichkeit gegenüber der Wasserhärte, ihre biologische Abbaubarkeit, ihre Verträglichkeit mit anderen Komponenten der Zubereitung sowie ihr Preis. Ein viel verwendetes Shampootensid ist beispielsweise Alkylethersulfat.

Neben Parfüm- und Farbstoffen sowie einer Reihe von Verbindungen, welche die Haltbarkeit der Zubereitungen erhöhen, werden in jüngerer Zeit den Haarshampoos unterschiedliche Arten von Wirkstoffen zugefügt. So ist der Zusatz von Silikonverbindungen bekannt, um dem Haar Glanz zu verleihen.

Der Glanz ist eine Eigenschaft, die dadurch zustande kommt, dass die Oberfläche eines Stoffes so glatt ist, dass Vertiefungen kleiner sind als die Wellenlänge des sichtbaren Lichtes. Der Glanz keratinischer Fasern hängt somit von der Oberflächenbeschaffenheit ab. Je rauer die Oberfläche (Cuticula), desto größer ist der Anteil an diffus reflektierten Licht und desto geringer ist der Glanz der Faser.

Das Haar lässt sich grob in zwei wesentliche Bestandteile unterteilen: das Haarinnere (lat. Cortex) und die dachziegelförmig um das Innere gelegte äußere Umhüllung des Haares.

Die Cuticula besteht aus feinen, dachziegelartig geschichteten Schuppen, die verhornte Keratinozyten darstellen. Derart gleichmäßige Oberflächen findet man in der Regel an jungem Haar unmittelbar nach dem Austreten des Haares aus dem Haarschaft. Die Cuticula bietet dem Cortex einerseits mechanischen und chemischen Schutz vor äußeren Einflüssen, andererseits Schutz gegen UV-Strahlen; sie verhindert das Austrocknen des Haares und die Entstehung von Spliss. Ferner ist die Oberfläche auch verantwortlich für das visuelle Erscheinungsbild, für den Glanz des Haares.

Durch mechanische, chemische und umweltbedingte Einflüsse kommt es zur Veränderung und Schädigung des Haares, insbesondere der Cuticula. Die Cuticulaschuppen sind dann oft an den Enden abgebrochen und die einzelnen liegen nicht mehr eng an die Oberfläche an. Solches Haar zeigt wenig Glanz.

Literaturbekannte Methode zur Verbesserung des Glanzes der Haare nach dem Waschen, ist dem beim Waschen verwendeten Shampoo Diamantpartikel zuzusetzen (Siehe EP 2020223 A2).

Diamant ist eine der kristallisierten Modifikationen des Kohlenstoffs. Er ist bei Oberflächenbedingungen gegenüber Graphit eigentlich thermodynamisch metastabil. Diamant hat eine Dichte von 3,52 $t/m^3$ und eine Mohs-Härte von 10, er ist damit das härteste irdische Material. Diamanten können im Stahlmörser zu Pulver zerstampft werden. Diamanten sind als hochwertige Schmucksteine bekannt. Eine höhere wirtschaftliche Bedeutung haben sie aber heute durch ihre industrielle Verwendung als Schneidstoffe und Schleifstoffe sowie als Zugabe in Polierpasten, wobei man sich ihre Härte und Verschleißfestigkeit zunutze macht.

Diamanten können auch synthetisch hergestellt werden. Dies gelang erstmals 1953/55. Die heutigen im thermodynamischen Stabilitätsbereich von Diamant operierenden Hochtemperatur-Hochdruck-Verfahren basieren auf der

Umwandlung von Graphit in Diamant bei 1200 - 1400°C und 5 - 7 GPa Druck unter Mitwirkung geschmolzener Metalle als Lösemittel und Katalysatoren, meist Eisen, Cobalt, Chrom, Aluminium und Titan, früher auch Nickel. Dabei können durch Optimierung der Züchtungsbedingungen wie chemische Reinheit des eingesetzten Kohlenstoffs (z.B. $^{12}C$) und geeignete Zusammensetzungen der metallischen Flussmittel Stickstoff-arme Kristalle mit verbesserten optischen Eigenschaften hergestellt werden. 1990 wurde ein synthetischer Diamantkristall von 14,2 Karat Gewicht hergestellt. Industriell übliche Synthesen erfolgen mit dem Kohlenstoff-Isotop $^{12}C$. Seit 1991 können farblose, lupenreine Diamanten mit 3 Karat Gewicht aus 99% $^{13}C$ hergestellt werden.

Mit dem Verfahren der CVD-Synthese (englisch chemical vapour deposition) lassen sich Diamanten auch bei Atmosphärendruck aus ~1000°C heißen Gasen (Methan oder andere Kohlenwasserstoffe) in Gegenwart eines Wasserstoff-Überschusses abscheiden. Dabei entsteht ein nanometer- bis mikrometerdünner metastabiler Diamantfilm auf Metallsubstraten.

Nanokristalline Diamanten können durch intensive Bestrahlung von Graphit erhalten werden. Dabei entstehen zunächst zwiebelartige Schalenstrukturen aus Kohlenstoff-Atomen, die bei fortgesetzter Bestrahlung Bindungen zwischen den Schalen bilden und damit eine Selbstkompression verursachen, bei der Diamantkristallite gebildet werden. Mit einer laserbeheizten transparenten Diamantstempelzelle ist es heute möglich, die Druck-Temperatur-Bedingungen im Erdkern zu simulieren.

Zum Glätten und zur Glanzvermittlung der Haaroberfläche wurden bisher kosmetische Zubereitungen eingesetzt, in denen zumeist Silikone oder pflegende Polyquaternium-Polymere eingesetzt werden, die eine hohe Affinität zum Haar haben und auf die Haaroberfläche aufziehen und diese dadurch glätten.

Nachteilig ist jedoch der Umstand, dass silikonhaltige Formulierungen von Verbrauchern weniger gewünscht werden. Auch ist der Einsatz von Polymeren, welche Homo- oder Copolymerisation mit Acrylsäure oder Methacrylsäure, sowie von Polyquaternium-Polymeren, wie u.a. Polyquaternium-10, von Verbrauchern nicht mehr erwünscht, da die biologische Abbauarbeit nicht abschließend geklärt ist.

Weiterhin ist der Einsatz von Polyethylenglykolhaltigen Verdickern, wie PEG-200 Hydrogenated Glyceryl Palmate oder PEG-7 Glycerylcocoat weitreichend verbreitet. So ist es oftmals möglich mit diesen partikelförmige Inhaltsstoffe in Schwebe im Produkt zu halten.

Jedoch ist auch der Einsatz von Polyethylenglykolhaltigen Verdickern zunehmend vom Verbraucher ungewünscht.

Somit steht der Fachmann vor dem Problem Produkte bereitzustellen, die eine ausreichende Viskosität aufweisen, so dass sie sich nicht zu schnell oder zu langsam aus handelsüblichen Packmitteln in Form von Flaschen mit ca. 2 mm großen Ausgabeöffnungen dosieren lassen. Die Erforderlichen Viskositäten liegen im Bereich von 3000 mPas bis 5000 mPas.

Problematisch ist es die Viskosität im dosierbaren Flaschenbereich unter Verzicht auf Polyethylengylkol-haltige Verdicker sowie unter Verzicht von Polymeren, welche Homo- oder Copolymerisation mit Acrylsäure oder Methacrylsäure einzustellen und gleichzeitig über den Verbrauchszeitraum des Produkts eine gleichmäßige "Glanzwirkung" des Produkts zu gewährleisten. So ist es insbesondere bei Diamantpartikeln problematisch, dass diese bei Lagerung in vielen Verdickersystemen sich am Boden der Produkte ablagern können. Entsprechend wird eine unzureichende Glanzleistung nach dem Waschen mit dem Shampoo Produkt erreicht. Dieses Problem tritt nicht bei Formulierungen auf, welche Silikone zur Glanzerzeugung enthalten, da die Silikone in der Formel zumeist gelöst vorliegen. Weiterhin ist es oftmals problematisch, dass unzureichende Glanzleistungen erreicht werden, wenn die Formulierungen keine Zusätze enthalten, die die Ablagerung von Diamantpartikeln auf den Haaren fördern. Im Stand der Technik werden zumeist Polyquaternium-Polymere oder Polymere, welche Homo- oder Copolymerisation mit Acrylsäure oder Methacrylsäure eingesetzt, die ein Aufziehen der Formel und somit der Diamantpartikel auf die Haare verbessern.

Überraschend konnte nun eine Shampoozubereitung bereitgestellt werden, welche die oben beschriebenen Nachteile und Probleme nicht aufweist.

Gegenstand der vorliegenden Erfindung ist eine Shampoozubereitung enthaltend, bezogen auf das Gesamtgewicht der Zubereitung,

a) Mindestens ein anionisches Tensid,
b) Mindestens ein ampotheres Tensid,
c) 0,0001 bis 0,1 Gew.-% Diamantpartikel,
d) 0,4 bis 3 Gew.-% Natriumchlorid,

dadurch gekennzeichnet, dass die Zubereitung frei von Polyethylenglykolhaltigen Verdickern ist.

Insbesondere Gegenstand der Erfindung ist eine Shampoozubereitung enthaltend, bezogen auf das Gesamtgewicht der Zubereitung,

a) Mindestens ein anionisches Tensid,

b) Mindestens ein ampotheres Tensid,

c) 0,0001 bis 0,1 Gew.-% Diamantpartikel,

d) 0,4 bis 3 Gew.-% Natriumchlorid,

dadurch gekennzeichnet, dass die Zubereitung frei von PEG-7 Glycerylcocoate und/oder Peg-200 Hydrogenated Glyceryl Palmate ist.

Ein weiterer Gegenstand ist die Verwendung der erfindungsgemäßen Shampoozubereitung zur Erhöhung des Glanzes der Haare nach dem Waschen der Haare mit der Shampoozubereitung und/oder zur Stabilisierung der Diamantpartikel in Schwebe in der Shampoozubereitung.

Sollten nachfolgend Gewichtsprozentangaben (Gew.-%) ohne Bezugnahme auf eine bestimmte Zusammensetzung oder spezifische Mischung angegeben werden, so beziehen sich diese Angaben immer auf das Gesamtgewicht der Zubereitung. Sollten nachfolgend Verhältnisse von Komponenten/Substanzen/Stoffgruppen offenbart werden, so beziehen sich diese Verhältnisse auf Gewichtsverhältnisse der genannten Komponenten/ Substanzen/Stoffgruppen.

Werden nachfolgend Gewichtsprozentbereiche für die Bestandteile der Zubereitung angegeben, so umfasst die Offenbarung der vorliegenden Anmeldung ebenfalls alle Einzelwerte in Schritten von 0,1 Gew.-% innerhalb dieser Gewichtsprozentbereiche.

Die Formulierungen "erfindungsgemäß", "erfindungsgemäß vorteilhaft", "vorteilhaft im Sinne der vorliegenden Erfindung" etc. beziehen sich im Rahmen der vorliegenden Offenbarung immer sowohl auf die erfindungsgemäße Zubereitung sowie die erfindungsgemäße Verwendung und das Verfahren.

Sofern nicht anders angegeben wurden alle Versuche unter Normalbedingungen durchgeführt. Der Begriff "Normalbedingungen" bedeutet 20°C, 1013 hPa und eine relative Luftfeuchtigkeit von 50%.

Wird der Begriff Haut verwendet, so bezieht dieser sich bevorzugt auf die menschliche Haut.

Erfindungsgemäß wird unter Polyethylenglykolhaltigen Verdickern Substanzen verstanden, welche mindestens eine Polyethylenglykoluntereinheit besteht mindestens aus 2 aufeinanderfolgenden Ethylenglykoleinheiten aufweist.

Der Begriff "frei von" bedeutet im Sinne der vorliegenden Offenbarung, dass der Anteil der genannten Substanz 0,1 Gew.-% nicht übersteigt und insbesondere 0 Gew.-%, bezogen auf das Gesamtgewicht der Zubereitung beträgt. Somit soll ein versehentliches Einschleppen durch Verunreinigungen nicht zum Ausschluss aus dem Schutzbereich führen.

Unter Emulgatoren werden alle Substanzen verstanden, welche im International Cosmetic Ingredient Dictionary and Handbook, Thirteenth Edition 2010, (ISBN 1-882621-47-6) unter der Bezeichnung "emulsifying agent" geführt werden. Unter Tensiden werden alle Substanzen verstanden, welche im International Cosmetic Ingredient Dictionary and Handbook, Thirteenth Edition 2010, (ISBN 1-882621-47-6) unter der Bezeichnung " surfactant " geführt werden.

Werden in dieser Offenbarung Viskositätswerte angegeben, so beziehen sich alle Werte auf eine Messung bei 25°C in einer 150 ml Weithalsflasche (VWR Nr.: 807-001) mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messsystems wird die Viskosität berechnet. Als Messkörper wird der Messkörper Nr.1 (Artikelnr. 200 0191), geeignet für einen Viskositätsbereich bis 10.000 [mPa s], Drehzahl Bereich 62,5 min-1, verwendet.

Werden in dieser Offenbarung Angaben zu einem Mittelwert gemacht, bezieht sich dieser Mittelwert immer auf arithmetische Mittel.

Werden in dieser Offenbarung Angaben zum Molekulargewicht von Polymeren gemacht, so beziehen diese sich auf eine Messung mittel Lichtstreuung.

Vorteilhafte anionische Tenside im Sinne der vorliegenden Erfindung sind

Acylaminosäuren und deren Salze, wie

- Acylglutamate, insbesondere Natriumacylglutamat
- Sarcosinate, beispielsweise Myristoyl Sarcosin, TEA-lauroyl Sarcosinat, Natriumlauroylsarcosinat und Natriumcocoylsarkosinat,

Sulfonsäuren und deren Salze, wie

- Acylisethionate, z.B. Natrium-/ Ammoniumcocoylisethionat,
- Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat, Dinatrium PEG-5 Laurylcitratsulfosuccinat und Derivate,

sowie Schwefelsäureester, wie

- Alkylethersulfat, beispielsweise Natrium-, Ammonium-, Magnesium-, MIPA-, TIPA-Laurethsulfat, Natriummyrethsulfat und Natrium $C_{12-13}$ Parethsulfat,
- Alkylsulfate, beispielsweise Natrium-, Ammonium- und TEA- Laurylsulfat.

Weitere vorteilhafte anionische Tenside sind

- Taurate, beispielsweise Natriumlauroyltaurat und Natriummethylcocoyltaurat,
- Ether-Carbonsäuren, beispielsweise Natriumlaureth-13 Carboxylat und Natrium PEG-6 Cocamide Carboxylat, Natrium PEG-7-Olivenöl-Carboxylat
- Phosphorsäureester und Salze, wie beispielsweise DEA-Oleth-10 Phosphat und Dilaureth-4 Phosphat,
- Alkylsulfonate, beispielsweise Natriumcocosmonoglyceridsulfat, Natrium $C_{12-14}$ Olefinsulfonat, Natriumlaurylsulfoacetat und Magnesium PEG-3 Cocamidsulfat.

Insbesondere bevorzugt ist Natriumlaurylethersulfat.

Erfindungsgemäß vorteilhaft umfasst die erfindungsgemäße Zubereitung anionische Tenside in einem Anteil von 8 bis 12 Gew.-% bezogen auf das Gesamtgewicht der Zubereitung.

Es ist insbesondere vorteilhaft, wenn Natriumlaurylethersulfat in einem Anteil von 8 bis 12 Gew.-%, bevorzugt von 8,5 bis 10 Gew.-%, bezogen auf das Gesamtgewicht der Zubereitung enthalten ist.

Geeignete amphotere und/oder zwitterionische Tenside können bevorzugt ausgewählt sein aus einer oder mehreren Verbindungen der nachfolgenden Formeln (I) bis (VII), in denen der Rest R für einen geradkettigen oder verzweigten, gesättigten oder ein- oder mehrfach ungesättigten Alkyl- oder Alkenylrest mit 7 bis 23 Kohlenstoffatomen (Formeln (I) und (II)) oder für einen geradkettigen oder verzweigten, gesättigten oder ein- oder mehrfach ungesättigten Alkyl- oder Alkenylrest mit 8 bis 24 Kohlenstoffatomen (Formeln (III) bis (VII)) steht:

(I) 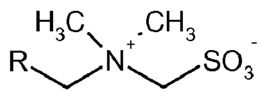

(II) 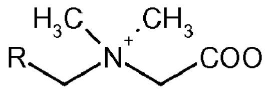

(III) 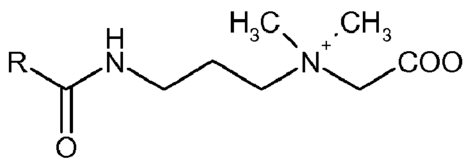

(IV) 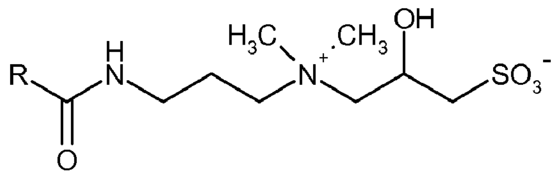

(V) 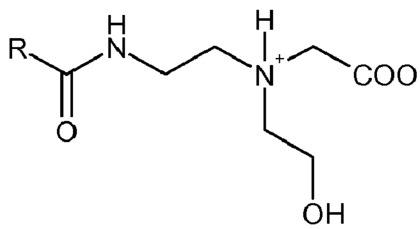

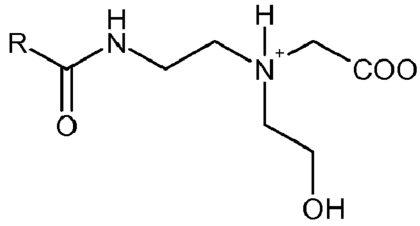

(VI)

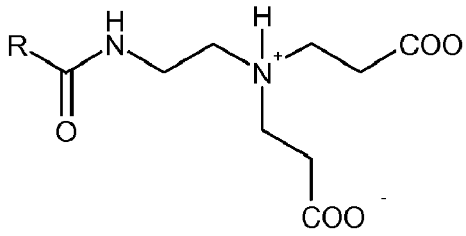

(VII)

Bevorzugte amphotere und/oder zwitterionische Tenside einer der zuvor genannten Formeln (I) bis (VII) enthalten als Rest R überwiegend einen geradkettigen oder verzweigten, gesättigten, ein- oder mehrfach ungesättigten Alkylrest mit 8 bis 20, mehr bevorzugt von 8 bis 16 und insbesondere mit 8 bis 12 C-Atomen.

Mehr bevorzugt sind amphotere und/oder zwitterionische Tenside, bei denen sich der Rest R von Kokosfett ableitet.

Insbesondere bevorzugt sind die unter den INCI-Bezeichnungen Sodium Cocoamphoacetate, Disodium Cocoamphodiacetate, Sodium Cocoamphopropionate, Disodium Cocoamphodipropionate, Coco Betaine, Lauryl Betaine und/oder Cocamidopropylbetain bekannten und im Handel von mehreren Anbietern erhältlichen amphoteren/zwitterionischen Tenside.

Es ist bevorzugt, wenn der Anteil der amphoteren Tenside von 1,5 bis 5,0 Gew.-%, bevorzugt von 2,5 bis 4 Gew.-%, bezogen auf das Gesamtgewicht der Zubereitung, beträgt.

Es ist insbesondere vorteilhaft, wenn Cocamidopropylbetain in einem Anteil von von 1,5 bis 5,0 Gew.-%, bevorzugt von 2,5 bis 4 Gew.-%, bezogen auf das Gesamtgewicht der Zubereitung, enthalten ist.

Weiterhin ist es vorteilhaft, wenn als zusätzliches Tensid ein Alkylglucosid enthalten ist.

Zusätzlich ist es vorteilhaft, wenn als Tensid mindestens ein Alkylglucosid, bevorzugt Decylglucoside enthalten ist. Das Decylglucosid wird vorteilhaft in Kombination mit Natriumlaurylethersulfat und Cocoamidopropylbetain eingesetzt.

Sofern ein Alkylglucosid, insbesondere Decylglucosid, enthalten ist, beträgt der Anteil von Alkylglucosid, insbesondere Decylglucosid, vorteilhaft von 0,1 bis 8 Gew.-%, bevorzugt von 0,5 bis 7 Gew.-% und insbesondere bevorzugt 0,75 bis 5 Gew.-%, bezogen auf das Gesamtgewicht der Zubereitung.

Es ist erfindungsgemäß weiterhin vorteilhaft, wenn die Diamantpartikel mit einer Teilchengröße von 1 bis 20 $\mu$m eingesetzt werden. Dieses entspricht der so genannten Diamant-Mikrokörnungen (erhältlich bei Firma Microdiamant). Als ganz besonders vorteilhaft hat sich eine mittlerer Teilchengröße von 4 - 6 $\mu$m gezeigt.

Die hier angegebene Teilchengröße bezieht sich auf den mittleren Durchmesser der Diamantpartikel. Unter dem "mittleren Durchmesser der Diamantpartikel" ist im Sinne der vorliegenden Erfindung bevorzugt der volumenmittlere Partikeldurchmesser D50 zu verstehen, der nach den üblichen Verfahren bestimmt werden kann, beispielsweise durch Laserdiffraktometrie.

Der volumenmittlere Partikeldurchmesser D50 ist derjenige Punkt in der Teilchengrößenverteilung, bei dem 50 Vol.-% der Diamantpartikel einen kleineren und 50 Vol.-% der Diamantpartikel einen größeren Durchmesser aufweisen.

Überraschend wurde gefunden, dass sich diese Diamantpartikel besonders gut in Schwebe halten lassen.

Weiterhin ist es vorteilhaft, wenn die Zubereitung Natriumchlorid umfasst, wobei es vorteilhaft ist, wenn der Anteil von Natriumchlorid von 0,45 bis 1,5 Gew.-%, bevorzugt von 0,45 bis 0,9 Gew.-% und insbesondere bevorzugt von 0,45 bis 0,8 Gew.-%, bezogen auf das Gesamtgewicht der Zubereitung, beträgt.

Weiterhin umfasst die Zubereitung vorteilhaft mindestens ein Guarhydroxypropyltrimoniumchlorid.

Dem Fachmann ist der Einsatz von Guarhydroxypropyltrimoniumchlorid in Shampoozubereitungen unter anderem aus EP1366742 bekannt.

Guarhydroxypropyltrimoniumchlorid ist ein Polymer, welches aus Guarmehl gewonnen werden kann. Abhängig von der gewollten Masse und Ladungsdichte kann das Syntheseverfahren angepasst werden. Mögliche Syntheseverfahren und die daraus erhaltenen Polymere mit gewünschter Masse und Ladungsdichte sind u.a. aus WO 2013/011122

A1 oder US4663159A bekannt.

Nachfolgend ist eine kurze Auswahl an Guarhydroxypropyltrimoniumchlorid Polymeren mit mittlerer Masse und Ladungsdichte angegeben:

Tab.1

| Handelsname | Mittlere Masse in Da | Ladungsdichte in meq/g |
|---|---|---|
| Jaguar® C-500 (Rhodia)[3] | 389 000 | 0,72 |
| Jaguar® C-17 (Rhodia)[3] | 2 000 000 | 1,04 |
| Jaguar® C 13S (Rhodia)[1] | 2 200 000 | 0,8 |
| Jaguar® C-14s (Rhodia)[3] | 2 000 000 | 0,72 |
| Jaguar® Optima (Rhodia)[2] | 500 000 | 1,3 |
| Jaguar® Excel (Solvay)[2] | 1 500 000 | 0,7 |
| Hi-Care 1000 (Rhodia)[1] | 600 000 | 0,7 |
| N-Hance 3270 (ASI/Ashland)[1] | 425 000 | 0,7 |
| N-Hance 3196 (ASI/Ashland)[1] | 1 100 000 | 0,8 |
| AquaCat CG518 (ASI/Ashland)[1] | 50 000 | 0,9 |
| Polymer 1 from WO 2013/011122 A1 | 333 000 | 1,25 |
| Polymer 2 from WO 2013/011122 A1 | 1 190 000 | 1,37 |
| Polymer 3 from WO 2013/011122 A1 | 305 000 | 1,17 |
| Polymer 4 from WO 2013/011122 A1 | 368 000 | 1,37 |
| [1]Bekannt aus EP 2999455 A1<br>[2]Bekannt aus US20180311135 A1<br>[3]Bekannt aus WO 2013/011122 A1 | | |

Der Begriff "Ladungsdichte", wie er hier verwendet wird, bezieht sich auf das Verhältnis positiver Ladungen an einer Monomereinheit, aus der ein Polymer besteht, zum Molekulargewicht der Monomereinheit. Die Ladungsdichte wird in meq/g (Milli-Äquivalente pro Gramm) angeben. Die Ladungsdichte multipliziert mit dem Molekulargewicht des Polymers bestimmt die Anzahl der positiv geladenen Stellen an einer gegebenen Polymerkette.

Der Begriff "kationische Gruppen", wie er hier verwendet wird, bezieht sich auf positiv geladene Gruppen und auf teilweise geladene Gruppen. Der Ausdruck "teilweise geladene Gruppen", wie er hier verwendet wird, bezeichnet Gruppen, die in Abhängigkeit vom pH-Wert der Formulierung positiv geladen werden können. Solche Gruppen können auch als "potentiell kationische Gruppen" bezeichnet werden. Wie hier verwendet, bedeutet der Begriff "kationisch" zumindest teilweise kationisch. So umfassen die Begriffe "kationisierende Mittel", "kationische Gruppen" und "kationische Einheiten" Ammonium (die eine positive Ladung aufweisen), aber auch primäre, sekundäre und tertiäre Amine und deren Vorläufer (die zu positiv geladenen Verbindungen führen können).

Messtechnisch kann für kationische Guars die Ladungsdichte unter Verwendung einer Standardelementaranalyse des prozentualen Stickstoffs gemessen werden, welche dem Fachmann bekannt ist. Bei den kationischen Copolymeren hängt die Ladungsdichte von den bei der Synthese verwendeten Monomeren ab. Dem Fachmann bekannte Standard-NMR-Techniken werden verwendet, um dieses Verhältnis von kationischen und nichtionischen Monomeren in dem Polymer zu bestätigen.

Die Zubereitung der vorliegenden Erfindung umfasst vorteilhaft ein erstes Guarhydroxypropyltrimethylammoniumchlorid. Dieses weist erfindungsgemäß vorteilhaft ein mittleres Molekulargewicht von 2000000 bis 5000000 Da, bevorzugt 2000000 bis 3500000 Da und insbesondere bevorzugt von 2000000 bis 2500000 Da auf.

Weiterhin ist es erfindungsgemäß vorteilhaft, wenn das erste Guarhydroxypropyltrimoniumchlorid dadurch gekennzeichnet ist, dass es eine Ladungsdichte von 0,5 bis 1,0 meq/g, bevorzugt von 0,6 bis 0,9 meq/g und insbesondere bevorzugt von 0,65 bis 0,85 meq/g.

Es ist weiterhin vorteilhaft, wenn der Anteil von dem ersten Guarhydroxypropyltrimoniumchlorid von 0,01 bis 0,5 Gew.-%, bevorzugt 0,03 bis 0,2 Gew.-% und insbesondere bevorzugt 0,06 bis 0,12 Gew.-%, bezogen auf das Gesamtgewicht der Zubereitung beträgt.

Die Zubereitung der vorliegenden Erfindung umfasst vorteilhaft ein zweites Guar-Guarhydroxypropyltrimoniumchlorid. Dieses weist vorteilhaft erfindungsgemäß ein mittleres Molekulargewicht von 400000 bis weniger als 2000000 Da, bevorzugt 600000 bis weniger als 1999999 Da und insbesondere bevorzugt von 1000000 bis 1900000 Da auf.

Weiterhin ist es erfindungsgemäß vorteilhaft, wenn das zweite Guarhydroxypropyltrimoniumchlorid dadurch gekennzeichnet ist, dass es eine Ladungsdichte von 0,5 bis 1,0 meq/g, bevorzugt von 0,6 bis 0,9 meq/g und insbesondere bevorzugt von 0,65 bis 0,85 meq/g aufweist.

Es ist weiterhin vorteilhaft, wenn der Anteil von dem zweiten Guarhydroxypropyltrimoniumchlorid von 0,01 bis 0,5 Gew.-%, bevorzugt 0,03 bis 0,2 Gew.-% und insbesondere bevorzugt 0,06 bis 0,12 Gew.-%, bezogen auf das Gesamtgewicht der Zubereitung, beträgt.

Vorteilhaft beträgt der Gesamtanteil von dem ersten und zweiten Guarhydroxypropyltrimoniumchlorid von 0,02 bis 0,3 Gew.-%, bevorzugt von 0,07 bis 0,2 Gew.-% und insbesondere bevorzugt von 0,1 bis 0,19 Gew.-%, bezogen auf das Gesamtgewicht der Zubereitung.

Weiterhin ist es vorteilhaft im Sinne der vorliegenden Erfindung, wenn das Gewichtsverhältnis vom ersten zum zweiten Guarhydroxypropyltrimoniumchlorid von 2:1 bis 1:2, bevorzugt 1,5:1 bis 1:1,5 und insbesondere bevorzugt von 1,2:1 bis 1:1,2 beträgt.

Weiterhin ist es vorteilhaft, wenn die Zubereitung weitere kationische Polymere in Anteilen von weniger als 0,2 Gew.-%, bevorzugt weniger als 0,1 Gew.-% und insbesondere bevorzugt weniger als 0,05 Gew.-% und insbesondere bevorzugt von 0 Gew.-% enthält. Folglich ist es am bevorzugtesten, wenn keine weiteren kationischen Polymere enthalten sind.

Weiterhin ist es vorteilhaft, wenn die Zubereitung weitere Polymere, aus genommen von Alkylglycosiden, in Anteilen von weniger als 0,2 Gew.-%, bevorzugt weniger als 0,1 Gew.-% und insbesondere bevorzugt weniger als 0,05 Gew.-% und insbesondere bevorzugt von 0 Gew.-% enthält.

Weiterhin ist es vorteilhaft, wenn die Zubereitung frei von Silikonverbindungen ist. Folglich beträgt der Anteil von Silikonverbindungen in der Zubereitung vorteilhaft von weniger als 0,1 Gew.-%, bevorzugt weniger als 0,05 Gew.-% und insbesondere bevorzugt 0 Gew.-%, bezogen auf das Gesamtgewicht der Zubereitung.

Weiterhin ist es vorteilhaft, wenn die Zubereitung frei von Polymeren aus Homo- oder Copolymerisation mit Acrylsäure und/oder Methacrylsäure ist. Folglich beträgt der Anteil von Polymere aus Homo- oder Copolymerisation mit Acrylsäure und/oder Methacrylsäure in der Zubereitung vorteilhaft von weniger als 2 Gew.-%, bevorzugt weniger als 1 Gew.-%, bevorzugt weniger als 0,5 Gew.-%, bevorzugt weniger als 0,2 Gew.-%, bevorzugt weniger als 0,1 Gew.-%, bevorzugt weniger als 0,05 Gew.-% und insbesondere bevorzugt 0 Gew.-%, bezogen auf das Gesamtgewicht der Zubereitung.

Weiterhin ist es vorteilhaft, wenn die Zubereitung frei von Polyquaternium Polymeren, insbesondere frei von Polyquaternium-7 und Polyquaternium-10, ist.

Weiterhin ist es vorteilhaft, wenn die Zubereitung frei von weiteren chemisch modifizierten katonischen Polymeren ist. Folglich beträgt der Anteil von weiteren chemisch modifizierten katonischen Polymeren in der Zubereitung vorteilhaft von weniger als 2 Gew.-%, bevorzugt weniger als 1 Gew.-%, bevorzugt weniger als 0,5 Gew.-%, bevorzugt weniger als 0,2 Gew.-%, bevorzugt weniger als 0,1 Gew.-%, bevorzugt weniger als 0,05 Gew.-% und insbesondere bevorzugt 0 Gew.-%, bezogen auf das Gesamtgewicht der Zubereitung.

Weiterhin ist es vorteilhaft, wenn die Zubereitung Sodium Benzoate umfasst. Sofern Sodium Benzoate enthalten ist, ist es weiterhin vorteilhaft, wenn der Anteil von Sodium Benzoate von 0,1 bis 0,8 Gew.-%, bevorzugt von 0,2 bis 0,7 Gew.-% und insbesondere bevorzugt von 0,3 bis 0,7 Gew.-%, bezogen auf das Gesamtgewicht der Zubereitung, beträgt.

Weiterhin ist es vorteilhaft, wenn die Zubereitung Zitronensäure umfasst. Sofern Zitronensäure enthalten ist, ist es weiterhin vorteilhaft, wenn der Anteil von Zitronensäure von 0,3 bis 1,5 Gew.-%, bevorzugt von 0,5 bis 1,3 Gew.-% und insbesondere bevorzugt von 0,7 bis 1,0 Gew.-%, bezogen auf das Gesamtgewicht der Zubereitung, beträgt.

Weiterhin ist es vorteilhaft, wenn die Zubereitung Natriumcitrat umfasst. Sofern Natriumcitrat enthalten ist, ist es weiterhin vorteilhaft, wenn der Anteil von Natriumcitrat von 0,1 bis 0,8 Gew.-%, bevorzugt von 0,2 bis 0,7 Gew.-% und insbesondere bevorzugt von 0,3 bis 0,7 Gew.-%, bezogen auf das Gesamtgewicht des Konzentrats, beträgt.

Es ist ebenfalls vorteilhaft im Sinne der Erfindung, wenn die Zubereitung eine Mikroemulsion umfasst. Vorteilhaft umfass die Microemulsion 10 bis 20 Gew.% Alkylpolyglykoside, 4 bis 20 Gew.% Monoester des Glycerins von C12-C22 Fettsäuren und 5 bis 30 Gew.% Ölkörper aufweist, wobei sich die Angaben auf das Gesamtgewicht der Mikroemulsion beziehen.

Die Mikroemulsion enthält als zwingende Bestandteile ein Alkylpolyglykoside, und zwar ein Alkyl(oligo)glycosid (im Folgenden auch als "APG" bezeichnet). Alkyl- und/oder Alkenyloligoglycoside im Sinne der vorliegenden Lehre folgen dabei der Formel $R^1O\text{-}[G]_p$ in der $R^1$ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligoglucoside. Die Indexzahl p in der allgemeinen Formel (I) gibt den Oligomerisierungsgrad (DP), d. h. die Verteilung von Mono- und Oligoglykosiden an und steht

für eine Zahl zwischen 1 und 10. Während p in einer gegebenen Verbindung stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,5 liegt. APGs sind in den Mikroemulsionen gemäß der vorliegenden Erfindung in Mengen zwischen 10 und 20 Gew.-%, jeweils bezogen auf die Gesamtmenge der Mikroemulsion enthalten. Besonders bevorzugt sind dabei Mengen im Bereich von 14 bis 19 Gew.-%, bezogen auf die Mikroemulsion. Insbesondere bevorzugt wird Decyl Glucoside als Alkylpolyglykoside eingesetzt.

Weiterhin sind Monoester aus Fettsäuren der Kettenlänge C12-C22 mit Glycerin in den Emulsionen enthalten. Dabei sind insbesondere Monoester von Glycerin mit ungesättigten linearen Fettsäuren geeignet. Besonders bevorzugt im Sinne der Erfindung ist Glycerinmonooleat. Diese Glycerinester sind in den Mikroemulsionen in Mengen von 4 bis 20 Gew.-%, vorzugsweise 8 bis 15 Gew.-% - jeweils bezogen auf das Gesamtgewicht der Mikroemulsion - enthalten.

Schließlich enthält die Mikroemulsion noch einen Ölkörper, also eine nichtwasserlösliche organische Phase in Mengen von 5 bis 30 Gew.-%. Dabei sind besonders bevorzugte Ölphasen ausgewählt aus der Gruppe von Guerbetalkoholen auf Basis von Fettalkoholen mit 6 bis 18 C-Atomen, Estern linearer C6-C22-Fettsäuren mit linearen oder verzweigten C6-C22-Fettalkoholen bzw. Estern von verzweigten C6-C13-Carbonsäuren mit linearen oder verzweigten C6-C22-Fettalkoholen, Ester von linearen C6-C22-Fettsäuren mit verzweigten Alkoholen, Estern von C6-C22-Fettalkoholen und/oder Guerbetalkoholen mit aromatischen Carbonsäuren, Triglyceriden auf Basis C6-C10-Fettsäuren, flüssigen Mono-/Di-/Triglyceridmischungen auf Basis von C6-C18-Fettsäuren, Estern von C2-C12-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzlichen Öle, verzweigten primäre Alkoholen, substituierten Cyclohexanen, linearen und verzweigten C6-C22-Fettalkoholcarbonaten, Guerbetcarbonaten auf Basis von Fettalkoholen mit 6 bis 18, vorzugsweise 8 bis 10 C Atomen, Estern der Benzoesäure mit linearen und/oder verzweigten C6-C22-Alkoholen, linearen oder verzweigten, symmetrischen oder unsymmetrischen Dialkylethern mit 6 bis 22 Kohlenstoffatomen pro Alkylgruppe, aliphatischen bzw. naphthenischen Kohlenwasserstoffe und/oder Dialkylcyclohexanen. Als Ölkomponente können jedoch auch feste Fette und/oder Wachse. Diese können auch in Mischung mit den im vorherigen Abschnitt genannten Ölen vorliegen. Typische Beispiele für Fette sind Glyceride, d.h. feste oder flüssige pflanzliche oder tierische Produkte, die im Wesentlichen aus gemischten Glycerinestern höherer Fettsäuren bestehen. Als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Tocopherole und ätherische Öle eignen sich ebenfalls als Ölkomponente. Insbesondere bevorzugt ist Dicaprylyl Ether als Ölkomponente.

Der Rest der Mikroemulsion umfasst auf 100 Gew.-% jeweils Wasser, wobei ggf ergänzende weitere Inhaltstoffe enthalten sein können.

EP2194956 B1 zeigt erfindungsgemäße Mikroemulsionen.

Der Anteil der Mikroemulsion in der Gesamtzubereitung beträgt vorteilhaft von 0,1 bis 2 Gew.-%, bevorzugt von 0,15 bis 0,5 Gew.-% und insbesondere bevorzugt von 0,2 bis 0,3 Gew.-%, bezogen auf das Gesamtgewicht der Zubereitung.

Es hat sich als besonders vorteilhaft herausgestellt, wenn die Komponenten der Mikroemulsion in Form einer Mikroemulsion zugegeben werden. Es ist weniger bevorzugt die Komponenten der Mikroemulsion ohne vorherige Emulgierung der Zubereitung zuzugeben.

Weiterhin ist es vorteilhaft, wenn die Zubereitung ein oder mehrere unter Normalbedingungen flüssige Parfümstoffe umfasst. Es ist bevorzugt, wenn der Anteil der flüssigen Parfümstoffe nicht mehr als 5 Gew.-% bezogen auf das Gesamtgewicht der Zubereitung, ausmacht. Vorteilhaft beträgt der Anteil der flüssigen Parfümstoffe von 0,01 bis 1,5 Gew.-%, bezogen auf das Gesamtgewicht der Zubereitung.

Die Messung des Glanzes des Haares nach Anwendung der erfindungsgemäßen Shampoozubereitung erfolgte wie folgt:

Sieben Haarsträhnen wurden mit einem Standardshampoo vorgewaschen und konditioniert. Anschließend wurden die trockenen Haarsträhnen mit dem Standard-Glanzpräparationsverfahren präpariert, nass auf Glanzboxrollen aufgewickelt und zur Bestimmung von t0 gemessen. Anschließend wurden die Stränge mit der erfindungsgemäßen Shampoozubereitung behandelt, nass auf Glanzboxrollen aufgewickelt und zur Bestimmung von t1 vermessen. Zur Messung des Glanzes wurde eine Opsira shine Box mit den nachfolgenden Daten zur Messung der Reflexionseigenschaften von Haarsträhnen mit parallel ausgerichteter Haarfaser, montiert auf einem konvexen Probenhalter, verwendet. Es wurden jeweilis drei Reflektionsmessungen mit den unten angegeben Messwinkeln pro Haarsträhne durchgeführt.

| | |
|---|---|
| Software | Luster Client by Opsira Luca Tool 4.1.3009 |
| Messwinkel | 0°, 5°, 10° |
| Cylinder R | 37.5 |
| Cylinder Y | 41.902 |
| EPA | 455 |
| Quelle X | 500 |
| Quelle Y | -125 |
| Shine (DEG) | -7.64647 |

Bestimmung des Glanzes:

$$L = \frac{\sum Spiegel}{\sum diffus} \times \frac{HWB\ Std}{HWB\ Spiegel}$$

Umso höher der Glanzwert L ist, desto glänzender sind die Haare.

**Vergleichsversuche und Beispiele**

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

Tab.2

| **Inhaltstoffe** | **Bsp.1** |
|---|---|
| Aqua | Ad 100 |
| Cocamidopropyl Betaine | 2,94 |
| Sodium Chloride | 0,7 |
| Glycerin | 0,252 |
| Sodium Laureth Sulfate | 9,00 |
| Diamond Powder | 0,001 |
| Decyl Glucoside | 1,52 |
| Coco Glucoside | 1,50 |
| Hydrogenated Castor Oil | 0,17 |
| Guar Hydroxypropyltrimonium Chloride** | 0,09 |
| Guar Hydroxypropyltrimonium Chloride*** | 0,13 |
| Mikroemulsion aus 52,5% Aqua, 0,5% Benzoic Acid, 2% Citric Acid, 15% Decyl Glucoside; 20% Dicaprylyl Ether und 10% Glyceryl Oleate | 0,75 |
| Sodium Citrate | 0,35 |
| Citric Acid | 1,05 |
| Glycerin | 0,09 |
| Glycol Distearate | 0,7 |
| Sodium Benzoate | 0,45 |

(fortgesetzt)

| Inhaltstoffe | Bsp.1 |
|---|---|
| Parfum | 0,55 |
| **Kommerzielles Guarhydroxypropyltrimoniumchlorid aufweisend eine Masse im Bereich von mehr als 2,0 Millionen Dalton bis 2,5 Millionen Dalton und eine Ladungsdichte von 0,75 meq/g.<br>***Kommerzielles Guarhydroxypropyltrimoniumchlorid aufweisend eine Masse im Bereich von mehr als 1,2 Millionen Dalton bis weniger als 2,0 Millionen Dalton und eine Ladungsdichte von 0,72 meq/g. | |

Die Rezeptur Bsp.1 wurde hergestellt. Die Glanzwertmessung ergab, dass bei Messung von 6 separaten Haarsträhnen der Glanzwert von 0,85 Glanzwert gemäß obigen Messungprotokoll auf 1,25 Glanzwert gemäß obigen Messungprotokoll zugenommen hat, nachdem die Haarsträhnen mit dem erfindungsgemäßen Shampoo gemäß Bsp.1 behandelt wurden. Vor der Messung wurden de Shampoos mit Wasser ausgespült. Die Standardabweichung für die Messungen betrug 0,11.

Weiterhin wurde überraschend gefunden, dass auch bei längerer Lagerung von 6 Monaten es zu keinen Ablagerungen von Diamantpartikeln am Boden gekommen ist.

Weitere Beispiele:

| Inhaltstoffe | Bsp.1 | Bsp. 2 | Bsp. 3 | Bsp. 4 | Bsp. 5 | Bsp. 6 |
|---|---|---|---|---|---|---|
| Aqua | Ad 100 | Ad 100 | Ad 100 | Ad 100 | Ad 100 | Ad 100 |
| Cocamidopropyl Betaine | 3,36 | 3,36 | 2,94 | 3,50 | 3,36 | 3,20 |
| Sodium Chloride | 0,504 | 0,504 | 0,44 | 0,53 | 0,504 | 0,48 |
| Glycerin | 0,252 | 0,252 | 0,22 | 0,26 | 0,252 | 0,24 |
| Sodium Laureth Sulfate | 9 | 8 | 9 | 10 | 8 | 9 |
| Decyl Glucoside | 1,50 | 2,00 | 2,00 | 1,00 | 2,00 | 1,50 |
| Guar Hydroxypropyltrimonium Chloride** | 0,09 | 0,09 | 0,09 | 0,09 | 0,135 | 0,09 |
| Guar Hydroxypropyltrimonium Chloride*** | 0,09 | 0,18 | 0,18 | 0,09 | 0,135 | 0,18 |
| Mikroemulsion aus 52,5% Aqua, 0,5% Benzoic Acid, 2% Citric Acid, 15% Decyl Glucoside; 20% Dicaprylyl Ether und 10% Glyceryl Oleate | 0,25 | 0,25 | 0,75 | 0,50 | 0,25 | 1,00 |
| Sodium Citrate | 0,35 | 0,35 | | 0,35 | | |
| Diamond Powder | 0,01 | 0,005 | 0,001 | 0,1 | 0,025 | 0,002 |
| Citric Acid | 0,9 | 0,8 | 0,75 | 0,90 | 0,7 | 0,75 |
| Sodium Benzoate | 0,6 | 0,6 | 0,6 | 0,6 | 0,6 | 0,6 |
| Sodium Chloride | 0,7 | 0,8 | 0,7 | 0,6 | 0,8 | 0,7 |
| Parfum | 0,7 | 0,6 | 0,5 | 0,5 | 0,7 | 0,7 |

## Patentansprüche

1. Shampoozubereitung enthaltend, bezogen auf das Gesamtgewicht der Zubereitung,

   a) Mindestens ein anionisches Tensid,
   b) Mindestens ein ampotheres Tensid,

c) 0,0001 bis 0,1 Gew.-% Diamantpartikel,
d) 0,4 bis 3 Gew.-% Natriumchlorid,

**dadurch gekennzeichnet, dass** die Zubereitung frei von Polyethylenglykolhaltigen Verdickern ist.

2. Zubereitung nach Anspruch 1 **dadurch gekennzeichnet, dass** anionische Tenside in einem Anteil von 8 bis 12 Gew.-% bezogen auf das Gesamtgewicht der Zubereitung enthalten sind.

3. Zubereitung nach einem der vorhergehenden Ansprüche **dadurch gekennzeichnet, dass** Natriumlaurylethersulfat als anionisches Tensid enthalten ist.

4. Zubereitung nach einem der vorhergehenden Ansprüche **dadurch gekennzeichnet, dass** der Anteil der amphoteren Tenside von 1,5 bis 5,0 Gew.-%, bevorzugt von 2,5 bis 4 Gew.-%, bezogen auf das Gesamtgewicht der Zubereitung, beträgt.

5. Zubereitung nach einem der vorhergehenden Ansprüche **dadurch gekennzeichnet, dass** Sodium Cocoamphoacetate, Disodium Cocoamphodiacetate, Sodium Cocoamphopropionate, Disodium Cocoamphodipropionate, Coco Betaine, Lauryl Betaine und/oder Cocamidopropylbetain als amphotere Tenside enthalten sind.

6. Zubereitung nach einem der vorhergehenden Ansprüche **dadurch gekennzeichnet, dass** Alkylglucoside enthalten sind, wobei der Anteil der Alkylglucoside von von 0,1 bis 8 Gew.-%, bevorzugt von 0,5 bis 7 Gew.-% und insbesondere bevorzugt 0,75 bis 5 Gew.-%, bezogen auf das Gesamtgewicht der Zubereitung, beträgt.

7. Zubereitung nach einem der vorhergehenden Ansprüche **dadurch gekennzeichnet, dass** die Diamantpartikel eine Teilchengröße von 1 bis 20 μm aufweisen.

8. Zubereitung nach einem der vorhergehenden Ansprüche **dadurch gekennzeichnet, dass** der Anteil von Natriumchlorid von 0,45 bis 1,5 Gew.-%, bevorzugt von 0,45 bis 0,9 Gew.-% und insbesondere bevorzugt von 0,45 bis 0,8 Gew.-%, bezogen auf das Gesamtgewicht der Zubereitung, beträgt.

9. Zubereitung nach einem der vorhergehenden Ansprüche **dadurch gekennzeichnet, dass** die Zubereitung mindestens ein Guarhydroxypropyltrimoniumchlorid umfasst.

10. Zubereitung nach einem der vorhergehenden Ansprüche **dadurch gekennzeichnet, dass** die Zubereitung weitere kationische Polymere in Anteilen von weniger als 0,2 Gew.-%, bevorzugt weniger als 0,1 Gew.-% und insbesondere bevorzugt weniger als 0,05 Gew.-% und insbesondere bevorzugt von 0 Gew.-% enthält.

11. Zubereitung nach einem der vorhergehenden Ansprüche **dadurch gekennzeichnet, dass** die Zubereitung frei von Polymeren aus Homo- oder Copolymerisation mit Acrylsäure und/oder Methacrylsäure ist.

12. Zubereitung nach einem der vorhergehenden Ansprüche **dadurch gekennzeichnet, dass** die Zubereitung frei von Polyquaternium Polymeren, insbesondere frei von Polyquaternium-7 und Polyquaternium-10, ist.

13. Zubereitung nach einem der vorhergehenden Ansprüche **dadurch gekennzeichnet, dass** die Zubereitung frei von Silikonverbindungen ist.

14. Zubereitung nach einem der vorhergehenden Ansprüche **dadurch gekennzeichnet, dass** die Zubereitung eine Mikroemulsion umfasst, welche vorteilhaft 10 bis 20 Gew.% Alkylpolyglykoside, 4 bis 20 Gew.% Monoester des Glycerins von C12-C22 Fettsäuren und 5 bis 30 Gew.% Ölkörper aufweist, wobei sich die Angaben auf das Gesamtgewicht der Mikroemulsion beziehen.

15. Verwendung einer Shampoozubereitung gemäß einem der vorhergehenden Ansprüche zur Erhöhung des Glanzes der Haare nach dem Waschen der Haare mit der Shampoozubereitung und/oder zur Stabilisierung der Diamantpartikel in Schwebe in der Shampoozubereitung.

Europäisches Patentamt
European Patent Office
Office européen des brevets

# EUROPÄISCHER RECHERCHENBERICHT

Nummer der Anmeldung
EP 23 15 1937

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D<br>Y | EP 2 020 223 A2 (BEIERSDORF AG [DE]) 4. Februar 2009 (2009-02-04)<br>* Absätze [0024], [0043], [0045], [0047], [0048]; Anspruch 13; Beispiele 2-2,2-4,4,4-2,5 * | 1-13,15<br>1-15 | INV.<br>A61K8/02<br>A61K8/19<br>A61K8/44<br>A61K8/46<br>A61K8/60<br>A61K8/73<br>A61Q5/02<br>A61Q5/12 |
| Y | DE 10 2007 033184 A1 (HENKEL AG & CO KGAA [DE]) 2. Juli 2009 (2009-07-02)<br>* Anspruch 7; Beispiele 5,21,34 * | 1-15 | |
| | | | RECHERCHIERTE SACHGEBIETE (IPC) |
| | | | A61K<br>A61Q |

Der vorliegende Recherchenbericht wurde für alle Patentansprüche erstellt

| Recherchenort | Abschlußdatum der Recherche | Prüfer |
|---|---|---|
| München | 11. Juli 2023 | Werner, Stefan |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

1

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 23 15 1937

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-07-2023

| Im Recherchenbericht angeführtes Patentdokument | Datum der Veröffentlichung | Mitglied(er) der Patentfamilie | Datum der Veröffentlichung |
|---|---|---|---|
| EP 2020223 A2 | 04-02-2009 | CN 101371814 A | 25-02-2009 |
| | | DE 102007037122 A1 | 05-02-2009 |
| | | DE 202008013431 U1 | 18-06-2009 |
| | | EP 2020223 A2 | 04-02-2009 |
| DE 102007033184 A1 | 02-07-2009 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 2020223 A2
- WO 2013011122 A1
- US 4663159 A
- EP 2999455 A1
- US 20180311135 A1
- EP 2194956 B1


### In der Beschreibung aufgeführte Nicht-Patentliteratur


- International Cosmetic Ingredient Dictionary and Handbook. 2010